# EUROPEAN PATENT APPLICATION

(11) **EP 1 477 193 A1**
(43) Date of publication of application: **17.11.2004**
(21) Application number: 03704715.6
(22) Date of filing: 21.01.2003
(51) Int. Cl.: A61M 1/00

(54) **SELF-DESTRUCTING SYRINGE**

(30) Priority: 21.01.2002 ES 200200136 U
(71) Applicant: Gloves Shamrock Line, S.L., 46727 Real De Gandia (ES)
(72) Inventor: CANADAS SERRANO, Luis, E-46680 Algemesi (ES)
(74) Representative: Urizar Anasagasti, José Antonio
(86) International application number: PCT/ES2003/000028
(87) International publication number: WO 2003/059411

(57) **Abstract**

The invention relates to a self-destructing syringe. The purpose of the inventive syringe is to prevent subsequent re-use once it has been used. Said syringe comprises a plunger (9) which can be broken in the lower part (4) thereof. The upper part (3) of the aforementioned plunger (9) comprises a cavity which is used to house a needle therein. The body (8) of the syringe is provided with a series of projecting elements (1) which are used to fix the cylindrical part (7) or cap in order to prevent needlestic accidents.

## Description

The object of this invention, as expressed in the descriptive paper, is a self-destructing syringe, which has been designed and developed with a view to the new innovations introduced therein, obtaining notable advantages in comparison with other already existing models.

The plunger, or inner piece, has a weakened or active-action part.

Inside the main body of the syringe is a ring which is thinner than the rest of the body and which is made of the same material as the syringe itself, which is to say plastic. This ring is located approximately 1 mm from the end of the main body, known as passive action. When the plunger comes into contact with said ring, this breaks the weakened area of the plunger. The idea is that contact between the active action and the passive action causes the plunger breaks making it impossible to re-use the syringe.

### Background of the invention

There is a series of syringes on the market that are designed to be disposable, which is to say, to be used only once, and which incorporate means of destruction or methods of blocking the plunger.

Some of the syringes currently in use have retractable needles.

Nevertheless, none of those already on the market include all the advances in just one syringe, and, what's more, they have passive rather than active safeties, which is to say, the safety must be activated by the user to ensure that these cannot be reused.

### A description of the invention

This invention incorporates certain innovations that provide a solution to the aforementioned disadvantages. The self-destructing syringe comprises a breakable plunger which can house a needle inside. It is made up of a cap-shaped cylindrical part to house the needle, and this piece has one or more longitudinal opposing cuts or grooves sufficiently large enough for the edges or projecting elements can slide along.

The cap-shaped cylindrical part has one or more longitudinal opposing cuts or grooves sufficiently large enough for the projecting elements of the cylindrical body of the self-destructing syringe to slide along.

The rod of the plunger has opposing projecting elements located at a specific distance from the end of the cylinder, thus permitting the piston of the plunger to move.

The piston of the plunger has opposing projecting elements and a weakened section so that the plunger will snap under the pressure caused by the projecting elements located inside the syringe, in such a way that this is trapped at the end of the syringe, making reuse impossible.

With the self-destructing syringe, one or more of the projecting elements located inside the main body serve to secure a cylindrical part with an internal cavity shaped like a cap to avoid accidental jabs with the needle.

To complete the description laid out below and in order to better understand the characteristics of the invention, this paper contains figures to aid understanding of the innovations and the advantages offered by the protection which is object this invention.

### A brief description of the drawings

Figure 1 shows the body of the syringe sectioned longitudinally and indicates the small edges or projecting elements on the outside and inside of the syringe.
Figure 2 shows the rod of the plunger as a whole and the rod of the sectioned part of the plunger, indicating the cavity or space where the needle is stored, and the weakened part of the rod of the plunger which is what allows the syringe to snap under pressure.
Figure 3 gives a partially sectioned view of the cylindrical part or cap, indicating the cuts or grooves, ranging from one to several, that can be found covering part of the length. Also indicated are the one or more transversal cuts or grooves that start off at the end one of the longitudinal cuts or grooves, making it easier to secure in place. The inner edge of one of its ends can also be observed.

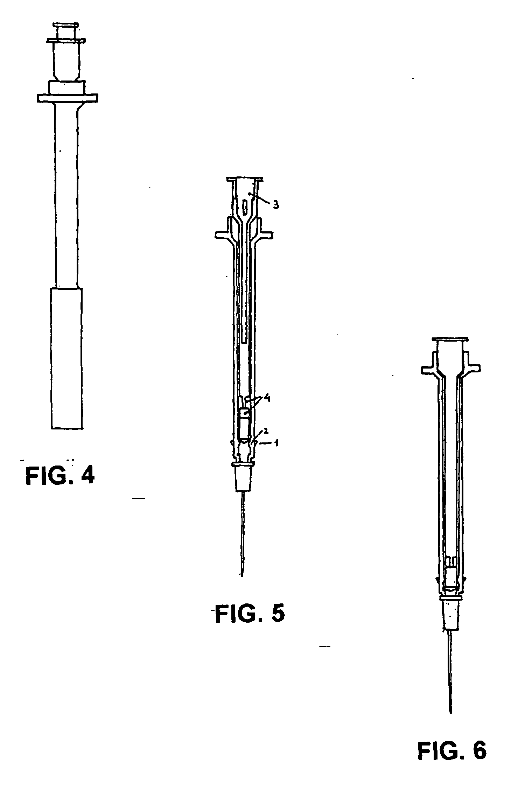

Figure 4 gives a view of the assembled syringe, with the needle in the upper part of the rod, and the cap or cylindrical part in the lower part of the syringe.
Figure 5 shows the syringe without the cap or cylindrical part and the needle in position for use. The rod of the plunger is located in the upper part so that the needle can be inserted and the breakable part to avoid reuse of the syringe can also be seen.
Figure 6 shows the position of the syringe once the injection has taken place, and the piston has finished its journey causing the plunger to snap, ruling out any further reuse.
Figure 7 shows the position of the syringe after the injection; the sectioned plunger can be removed and used to cover the needle instead of the cap.
Figure 8 gives a view of the syringe and shows where the needle is stored within the upper cavity of the rod of the plunger.

### Preferable use of the invention

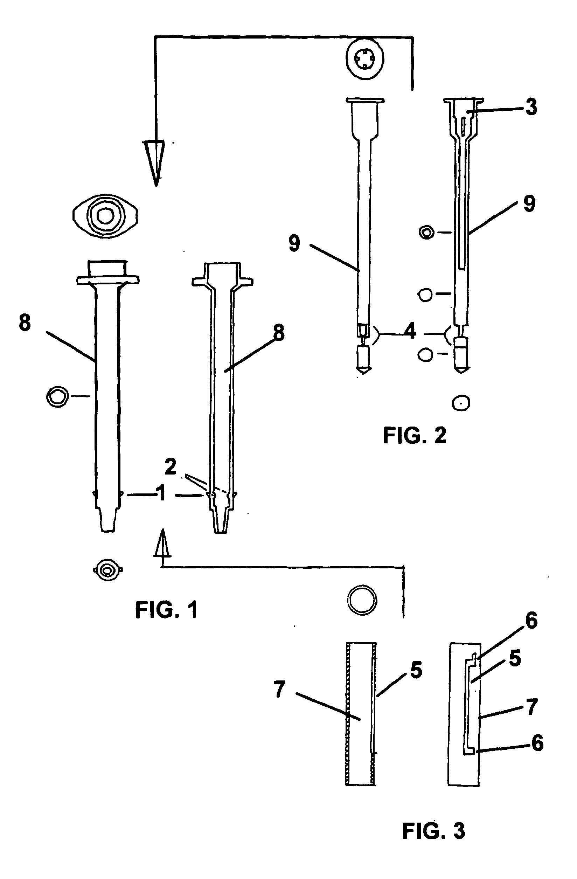

Figures 1, 2 and 3 show how this invention should preferably be used in practice, and indicates the configuration of the self-destructing syringe, where the parts adopt the form of a cylinder (7) or cap, which has one of more longitudinal cuts (5) or grooves, which are partially covered by small transversal cuts (6) or grooves, which begin at the end of cuts (5) or grooves in the cylindrical part.

The main body (8) of the syringe, which may or may not have the needle set in place and may or may not have a cover to protect said needle, has several small edges (1) or projecting elements to secure this to the cylindrical part (7) or cap.

The pieces that make up the self-destructing syringe include the rod (3) of the plunger, with its cavity in the upper part used to house the needle. Said needle can be inserted before or after the syringe is used. The rod has one part (4) that can be broken to ensure the syringe is not reused.

In order to use the self-destructing syringe, pull the cylindrical part (7) or cap backwards until it can go no further, and rotate the syringe slightly so that the small edges (1) or projecting elements are introduced into the cylindrical part (7) like a cap, thus facilitating use of the self-destructing syringe.

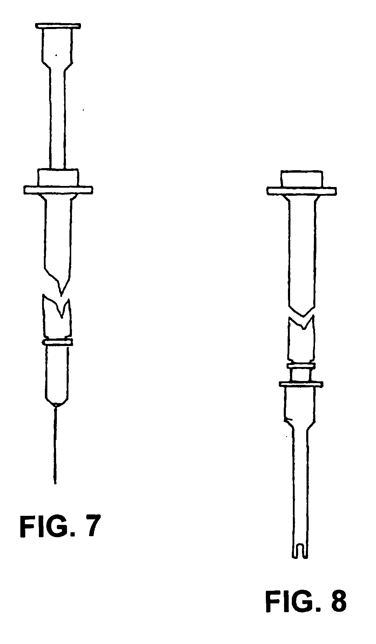

After use, and given the rod of the plunger has caused the syringe to break under pressure after use and has been released, this can be used to house or cover the needle as can be seen in figures 7 and 8.

This type of self-destructing syringe can be used with or without a cap, as it can be disposed of with no risk of further use.

As the nature of this invention has been sufficiently described, as has the way in which it should be used, all that remains is to add that the form, materials and way in which the syringe as a whole and its parts are arranged can be modified, provided said alterations do not considerably vary the characteristics to which the claims laid below refer.

## Claims

1. A self-destructing syringe the purpose of which is to prevent subsequent re-use. Said syringe comprises a cylindrical part (7) or cap, the body (8) of the syringe and a series of edges (1), or projecting elements on the outer wall of the body of the syringe, a breakable plunger (9), with a cavity (3) in the upper part to house the needle.

2. A self-destructing syringe, in accordance with the first claim, which is **characterized in that**, when pressure is exerted on the cylindrical body (7), or cap following the length of the body (8) of the syringe, said cylindrical body (7), or cap becomes anchored to the body (8) of the syringe itself, leaving the needle, or troncoconical part of the body of the syringe used to hold the needle is place, exposed.

3. A self-destructing syringe, in accordance with the first and second claim, which is **characterized in that** the plunger (9) can be broken, as the weakened area (4), much thinner that the rest of the syringe, will snap when the plunger (9) is put under pressure inside the body (8) of the syringe. The upper part (3) of the aforementioned plunger (9) comprises a cavity (3) used to house a needle therein, and said needle can be housed before or after used as shown on figure 8.

4. A self-destructing syringe, in accordance with the earlier claims, which is **characterized in that** the lower part of the inner body (8) of the syringe is provided with a series of projecting elements that serve to snap the plunger (9), before the injection has been finished, thus ensuring the syringe cannot be subsequently reused.
